# EUROPÄISCHE PATENTANMELDUNG

(11) **EP 4 119 108 A1**
(43) Veröffentlichungstag der Anmeldung: **18.01.2023**
(21) Anmeldenummer: 22184866.6
(22) Anmeldetag: 14.07.2022
(51) Int. Cl.: A61F 5/01

(54) **GELENKANORDNUNG FÜR EINE ORTHESE EINES EXTREMITÄTENGELENKS, SET ZUM KONFIGURIEREN DER GELENKANORDNUNG UND ORTHESE MIT EINER DERARTIGEN GELENKANORDNUNG**

(30) Priorität: 14.07.2021 DE 102021118201
(71) Anmelder: Bort GmbH, 71384 Weinstadt-Benzach (DE)
(72) Erfinder: Fischer, Klaus-Peter, 96528 Frankenblick (DE); Telles, Hans, 71384 Weinstadt (DE)
(74) Vertreter: Thum, Bernhard

(57) **Zusammenfassung**

Die Erfindung betrifft eine Gelenkanordnung (10) für eine Orthese eines Extremitätengelenks. Die Gelenkanordnung umfasst einen ersten Gelenkschenkel (12) mit einem länglichen ersten Schenkelabschnitt (14) und einem ersten Kopfabschnitt (16) und einen zweiten Gelenkschenkel (18) mit einem länglichen zweiten Schenkelabschnitt (20) und einem zweiten Kopfabschnitt (21). Der erste Gelenkschenkel (12) und der zweite Gelenkschenkel (18) überlappen sich im Bereich des ersten und des zweiten Kopfabschnitts (16, 21) und sind zueinander verschwenkbar. Der erste Kopfabschnitt (16) umfasst eine gekrümmte erste Führungsausnehmung (22), in die ein erster Führungskörper (38) eingreift, und eine von der ersten Führungsausnehmung (22) beabstandete zweite Führungsausnehmung (24), in die ein zweiter Führungskörper (36) eingreift. Während einem Verschwenken der Gelenkschenkel (12, 18) zueinander bewegt sich der erste Führungskörper (38) entlang einer durch die Innenkontur der ersten Führungsausnehmung (22) definierten ersten Bewegungsbahn relativ zu der ersten Führungsausnehmung (22), und bewegt sich der zweite Führungskörper (36) entlang einer zweiten Bewegungsbahn relativ zu der zweiten Führungsausnehmung (24). Die gekrümmte erste Führungsausnehmung (22) weist eine Umfangsfläche auf, die einen im Wesentlichen konkaven und einen im Wesentlichen konvexen Flächenabschnitt (25, 26) aufweist. Eine Längsachse der zweiten Führungsausnehmung (24) schneidet eine von dem ersten Schenkelabschnitt (14) definierte Längsachse (32) unter einem Winkel (β). Ferner stellt die Erfindung ein Set zum Konfigurieren der Gelenkanordnung und eine Orthese bereit, die eine derartige Gelenkanordnung umfasst.

## Beschreibung

Die vorliegende Erfindung betrifft eine Gelenkanordnung für eine Orthese eines Extremitätengelenks, ein Set zum Konfigurieren einer derartigen Gelenkanordnung und eine Orthese mit einer derartigen Gelenkanordnung.

Orthesen werden bekanntermaßen dazu eingesetzt, Bereiche eines Körpers eines Patienten zu stabilisieren, zu entlasten, zu führen oder hinsichtlich der Haltung oder Bewegung zu korrigieren. Dies kann therapeutisch im Rahmen einer Behandlung einer diagnostizierten Erkrankung des Patienten, zur Rehabilitation oder auch zur dauerhaften prophylaktischen Unterstützung des Patienten erfolgen. Im Falle einer Orthese, die am Körper eines Patienten einer Bewegung des Körpers ausgesetzt ist, wie beispielsweise einer Orthese für ein Extremitätengelenk, ist es vorteilhaft, wenn die eingesetzte Orthese einem möglichst natürlichen physiologischen Bewegungsablauf des Körpers bzw. des zu unterstützenden Extremitätengelenks folgt. Allerdings können Extremitätengelenke komplexe natürliche Bewegungsabläufe vollziehen. Derart komplexe Bewegungsabläufe liegen insbesondere dann vor, wenn der natürliche Bewegungsablauf des Extremitätengelenks keine eindeutige Drehachse aufweist, um die das Extremitätengelenk schwenkt. Gegebenenfalls können mehrere sich verändernde Drehbewegungen um verschiedene Drehachsen vorliegen, die sich mit linearen Bewegungsabläufen überlagern. Entsprechend anspruchsvoll ist es, einen solchen komplexen physiologischen Bewegungsablauf mit einer Orthese nachzubilden.

Eine Orthese umfasst üblicherweise Befestigungsmittel, mit denen sie an dem Extremitätengelenk bzw. das Extremitätengelenk überbrückend an dem Patienten befestigt werden kann. Bildet die Orthese des Extremitätengelenks allerdings nicht den natürlichen physiologischen Bewegungsablauf nach, so führt dies zu Nachteilen für den Patienten, die den Heilungserfolg sogar verzögern oder dem zu stützenden Gelenk auf Dauer schaden können. Die Befestigungsmittel der Orthese können sich beispielsweise relativ zu dem Patienten und insbesondere relativ zur Haut des Patienten bewegen, was zu unangenehmen Hautreizungen des Patienten führen kann. Insgesamt kann sich somit für einen Patienten ein unangenehmer Tragekomfort einstellen. Auch kann eine Orthese, die nicht den natürlichen physiologischen Bewegungsablauf des Extremitätengelenks nachbildet, dem Extremitätengelenk beispielsweise einen Bewegungsablauf auferlegen, der den physiologischen Bewegungsablauf hemmt. Dies kann zu einer Beeinträchtigung bis hin zu einer verstärkten Abnutzung des Extremitätengelenks oder auch zu einem unangenehmen Tragekomfort führen. Ferner kann dies weitere Krankheitsbilder begünstigen oder eine Rehabilitation erschweren.

Ein komplexer physiologischer Bewegungsablauf liegt beispielsweise im menschlichen Kniegelenk vor, denn dieses vollzieht zumindest phasenweise eine kombinierte Roll- und Gleitbewegung. Die kombinierte Roll- und Gleitbewegung betrifft im technischen Sinne Drehbewegungen eines Körpers. Eine Drehbewegung ist jedoch als Drehung eines Körpers um eine feststehende Drehachse spezifiziert. Eine Rollbewegung ist hingegen eine Drehung um eine sich bewegende Drehachse. So ist eine Rollbewegung vergleichbar mit einem Rad, das auf einer Ebene abrollt. Das Zentrum des Rades bewegt sich in der Richtung der Rollbewegung. Eine Gleitbewegung ist vergleichbar mit einem Rad, das auf der Ebene abrutscht. Das Zentrum des Rades verändert sich gegenüber der Ebene nicht. Im Fall der kombinierten Roll- und Gleitbewegung findet demnach eine Bewegung statt, bei der das Rad zwar auf der Ebene abrollt, sich jedoch die Drehachse um einen bestimmten Betrag in Richtung der Bewegung bewegt. Es hat sich in der Vergangenheit gezeigt, dass gerade die natürliche physiologische Bewegung eines Kniegelenks mechanisch mit einer Kniegelenk-Orthese nur äußerst schwer nachgebildet werden kann, insbesondere über den gesamten Beugungsbereich des Kniegelenks.

Aus dem Stand der Technik sind Versuche bekannt, komplexe physiologische Bewegungsabläufe von Extremitätengelenken besser nachzubilden. So ist beispielsweise aus dem Dokument EP 2 524 672 B1 eine Gelenkanordnung für eine Kniegelenk-Orthese bekannt. Die Gelenkanordnung umfasst einen Führungskörper, der mit einem ersten Gelenkschenkel verbunden ist, und eine Ausnehmung, die an einem zweiten Gelenkschenkel angeordnet ist. Der Führungskörper ist beispielsweise in Form eines Dreiecks ausgebildet und ist derart in der zugehörigen Ausnehmung geführt, die drei Führungskurven aufweist, dass er in der Ausnehmung und entlang der Führungskurven gleitet.

Ferner ist aus dem Dokument AT 510 403 A4 eine Gelenkanordnung für eine Kniegelenkorthese bekannt. Die Gelenkanordnung umfasst zwei Gelenkschenkel, die jeweils einen Kopfabschnitt aufweisen. Die Kopfabschnitte liegen aneinander an und sind miteinander gekoppelt. In einem ersten der Kopfabschnitte sind zwei Führungsbahnen in Form von Ausnehmungen vorgesehen, in die ein jeweiliger Führungskörper eingreift. Die Führungskörper sind mit einem zweiten der Kopfabschnitte gekoppelt. Diese Anordnung ermöglicht eine kombinierte Roll- und Gleitbewegung in der durch die beiden Gelenkschenkel definierten Schwenkebene. Die Drehachse bleibt stationär bei einem Verschwenken der beiden Gelenkschenkel zueinander, wenn der Beugewinkel der Gelenkschenkel zwischen 0° und 25° beträgt. Bei einem Beugewinkel größer als 25° verschiebt sich die Schwenkachse während eines fortschreitenden Verschwenkens der beiden Gelenkschenkel zueinander.

Aus dem Dokument WO 95/03013 A ist eine weitere Gelenkanordnung bekannt, die zwei Gelenkschenkel mit einem jeweiligen Kopfabschnitt umfasst. Ein erster der beiden Kopfabschnitte umfasst zwei bogenförmige Führungen, in die jeweils ein mit dem anderen Kopfabschnitt gekoppelter Bolzen eingreift. Ein zweiter der beiden Kopfabschnitte umfasst eine weitere bogenförmige Führung, in die ein Bolzen eingreift, der mit dem ersten Kopfabschnitt gekoppelt ist. Bei einer Beugebewegung erfolgt eine Relativbewegung aller drei Bolzen gegenüber den drei bogenförmigen Führungen. Ferner umfasst der zweite Kopfabschnitt Gewindebohrungen zum Einsetzen von Bolzen, die die Streckung oder Beugung der Gelenkanordnung begrenzen können.

Die aus dem Stand der Technik bekannten Orthesen mit Gelenkanordnung, die einen Bewegungsablauf mit einer sich ändernden Drehachse nachbilden sollen, sind allerdings mit Nachteilen verbunden. Trotz vieler Bemühungen wird es nicht oder allenfalls ansatzweise erreicht, den natürlichen physiologischen Bewegungsablauf des Extremitätengelenks tatsächlich nachzubilden. Häufig müssen teils wesentliche Vereinfachungen des natürlichen Bewegungsablaufs in Kauf genommen werden. Damit sind zumindest Phasen des natürlichen physiologischen Bewegungsablaufs unzureichend durch die Gelenkanordnung nachgebildet. Dies führt zu den genannten Problemen. Ferner weisen bekannte Gelenkanordnungen eine Vielzahl an Komponenten auf, was zu einer aufwändigen Fertigung führt.

Eine Aufgabe der vorliegenden Erfindung besteht daher darin, eine verbesserte Gelenkanordnung für eine Orthese eines Extremitätengelenks bereitzustellen.

Insbesondere ist es eine Aufgabe der vorliegenden Erfindung, eine gegenüber dem Stand der Technik verbesserte Gelenkanordnung für eine Orthese eines Extremitätengelenks bereitzustellen, die einen Bewegungsablauf mit sich veränderndem Drehzentrum realisiert, sodass der natürliche physiologische Bewegungsablauf des betreffenden Extremitätengelenks möglichst exakt nachgebildet werden kann.

Ferner ist es eine Aufgabe der Erfindung, ein Set zum Konfigurieren der Gelenkanordnung bereitzustellen, so dass die Gelenkanordnung an verschiedene Therapiestufen anpassbar ist.

Zudem besteht eine Aufgabe der Erfindung darin, eine Orthese mit einer verbesserten Gelenkanordnung bereitzustellen.

Die erfindungsgemäße Aufgabe wird durch eine Gelenkanordnung für eine Orthese eines Extremitätengelenks mit den Merkmalen des Anspruchs 1 gelöst. Ferner wird die erfindungsgemäße Aufgabe durch ein Set zum Konfigurieren der Gelenkanordnung mit den Merkmalen des Anspruchs 21 sowie durch eine Orthese mit den Merkmalen des Anspruchs 22 gelöst. Vorteilhafte Weiterbildungen der Erfindung finden sich in den Unteransprüchen.

Die oben genannte Aufgabe wird gemäß Anspruch 1 durch eine Gelenkanordnung für eine Orthese eines Extremitätengelenks gelöst. Die Gelenkanordnung umfasst einen ersten Gelenkschenkel mit einem länglichen ersten Schenkelabschnitt und einem ersten Kopfabschnitt sowie einen zweiten Gelenkschenkel mit einem länglichen zweiten Schenkelabschnitt und einem zweiten Kopfabschnitt. Der erste Gelenkschenkel und der zweite Gelenkschenkel überlappen sich im Bereich des ersten und des zweiten Kopfabschnitts zumindest teilweise und sind ferner derart gelenkig miteinander verbunden, dass die Gelenkschenkel in einer Schwenkebene zueinander verschwenkbar sind. Der erste Kopfabschnitt umfasst eine gekrümmte erste Führungsausnehmung, in die ein erster Führungskörper derart eingreift, dass dieser relativ zu der ersten Führungsausnehmung bewegbar ist. Ferner umfasst der erste Kopfabschnitt eine von der ersten Führungsausnehmung beabstandete zweite Führungsausnehmung, in die ein zweiter Führungskörper derart eingreift, dass dieser relativ zu der zweiten Führungsausnehmung bewegbar ist. Der erste Führungskörper ist mit dem zweiten Kopfabschnitt gekoppelt. Während einem Verschwenken der Gelenkschenkel zueinander bewegt sich der erste Führungskörper entlang einer durch die Innenkontur der ersten Führungsausnehmung definierten ersten Bewegungsbahn relativ zu der ersten Führungsausnehmung. Ferner bewegt sich der zweite Führungskörper entlang einer zweiten Bewegungsbahn relativ zu der zweiten Führungsausnehmung. Die gekrümmte erste Führungsausnehmung weist eine Umfangsfläche auf, die einen im Wesentlichen konkaven und einen im Wesentlichen konvexen Flächenabschnitt aufweist, wobei der im Wesentlichen konkave Flächenabschnitt der Umfangsfläche der ersten Führungsausnehmung dem ersten Schenkelabschnitt zugewandt ist und der im Wesentlichen konvexe Flächenabschnitt der Umfangsfläche der ersten Führungsausnehmung dem ersten Schenkelabschnitt abgewandt ist. Eine Längsachse der zweiten Führungsausnehmung schneidet eine von dem ersten Schenkelabschnitt definierte Längsachse unter einem Winkel, der vorzugsweise in einem Bereich zwischen 5° und 85° liegt.

Die Längsachse der zweiten Führungsausnehmung kann durch zwei Endanschläge der zweiten Führungsausnehmung verlaufen, die an einem ersten bzw. einem zweiten Ende der zweiten Führungsausnehmung angeordnet sein können. Die zwei Endanschläge können die zweite Bewegungsbahn des zweiten Führungskörpers begrenzen. Die zweite Führungsausnehmung kann eine beliebige Form aufweisen, solange diese die zwei Endanschläge definiert. Beispielsweise kann die zweite Führungsausnehmung eine längliche Form oder eine bohnenförmige Form aufweisen.

Durch die erfindungsgemäße Gelenkanordnung kann erreicht werden, dass ein vorgegebener Bewegungsablauf erzeugt wird, der der natürlichen physiologischen Bewegung des menschlichen Knies weitgehend exakt nachempfunden ist. Der Bewegungsablauf ist dabei durch die Anordnung und Ausgestaltung der ersten und zweiten Führungsausnehmung definiert. Ferner ermöglicht die Anordnung und Ausgestaltung der ersten und zweiten Führungsausnehmung die Gelenkschenkel relativ zueinander derart zu bewegen, dass ein großer Bereich von Beugewinkeln zwischen Längsachsen der Gelenkschenkel erzielt werden kann. Die Gelenkanordnung kann insbesondere eine Beugung des Extremitätengelenks von 0° bis zu 135°, vorzugsweise bis zu 140°, weiter bevorzugt bis zu 145°, nachbilden. Bei einem Beugewinkel von 0° sind die Längsachsen der Gelenkschenkel, genauer gesagt die Längsachsen des ersten und zweiten Schenkelabschnitts, zueinander parallel oder zusammenfallend ausgerichtet.

Die Führung des ersten Führungskörpers entlang der ersten Bewegungsbahn und des zweiten Führungskörpers entlang der zweiten Bewegungsbahn ermöglicht eine definierte Bewegung der Gelenkschenkel relativ zueinander, die mit wenigen Komponenten realisierbar ist.

Ferner wird durch die erfindungsgemäße Gelenkanordnung ein einfacher Aufbau erreicht, der den natürlichen Bewegungsablauf des Extremitätengelenks nachbildet, ohne dass an dem Bewegungsablauf eine Vielzahl an Komponenten beteiligt ist. Vorteilhafterweise kann ferner auftretende Reibung zwischen dem jeweiligen Führungskörper und der zugeordneten Ausnehmung reduziert werden. Damit kann eine gesteigerte Lebensdauer der erfindungsgemäßen Gelenkanordnung erreicht werden.

Ferner kann erfindungsgemäß vorgesehen sein, dass die Längsachse der zweiten Führungsausnehmung weder zu einer Längsachse des ersten Gelenkschenkels ausgerichtet ist noch mit dieser einen rechten Winkel bildet. Auf diese Weise kann eine der natürlichen physiologischen Kniebewegung nachempfundene Rollbewegung mit sich ändernder bzw. wandernder Drehachse erzielt werden.

Gemäß einer Weiterbildung der vorliegenden Erfindung kann vorgesehen sein, dass der erste Führungskörper mit einem seitlichen Bereich des zweiten Kopfabschnitts gekoppelt ist. Der erste Führungskörper definiert zusammen mit der ersten Führungsausnehmung die relative Bewegung des ersten und zweiten Kopfabschnitts. Eine Anordnung des ersten Führungskörpers im seitlichen Bereich des zweiten Kopfabschnitts ermöglicht eine möglichst große Längserstreckung der ersten Führungsausnehmung im Bereich des ersten Kopfabschnitts, wodurch ein möglichst großer Beugewinkel der Gelenkschenkel einstellbar ist. Somit kann eine die Gelenkanordnung umfassende Orthese die Beugung des Extremitätengelenks, beispielsweise eines Knies, bis zu einem großen Beugewinkel von beispielsweise zwischen 135° und 145°, vorzugsweise etwa 140°, unterstützen.

Ferner kann erfindungsgemäß vorgesehen sein, dass der zweite Führungskörper mit einem zentralen Bereich des zweiten Kopfabschnitts gekoppelt ist. Beispielsweise kann der zweite Führungskörper den zentralen Bereich des zweiten Kopfabschnitts durchsetzen. Dies begünstigt die Stabilität der Gelenkanordnung, da während einer Beugung der Gelenkschenkel die beiden Kopfabschnitte einen großen Überlappungsbereich aufweisen.

Für eine möglichst stabile Gelenkanordnung kann zudem beispielsweise die zweite Führungsausnehmung näher an dem ersten Schenkelabschnitt angeordnet sein als die gekrümmte erste Führungsausnehmung. Eine Ausgestaltung, bei der die gekrümmte erste Führungsausnehmung mindestens doppelt so lang, bevorzugt mindestens dreimal so lang, ist wie die zweite Führungsausnehmung begünstigt einen großen Beugewinkel zwischen den Längsachsen der Gelenkschenkel.

Gemäß einem weiteren Aspekt der Erfindung kann die Gelenkanordnung ferner ein Kopplungselement umfassen, mittels dem der erste Kopfabschnitt und der zweite Kopfabschnitt aneinander befestigt sind. Das Kopplungselement befestigt die beiden Kopfabschnitte aneinander, sodass diese einen definierten Abstand zueinander in Bezug auf deren Schwenkebene aufweisen. Zudem erlaubt das Kopplungselement eine flache und damit kompakte Ausgestaltung der Gelenkanordnung.

Gemäß einer Weiterbildung der vorliegenden Erfindung kann vorgesehen sein, dass das Kopplungselement mit dem zweiten Führungskörper und einem Kopplungskörper gekoppelt ist, der mit dem ersten Kopfabschnitt gekoppelt ist. Die Kopplung des Kopplungskörpers mit dem ersten Kopfabschnitt kann eine ortsfeste Kopplung sein. Beispielsweise ist der Kopplungskörper in einer Kopplungsausnehmung des ersten Kopfabschnitts spielfrei oder spielarm aufgenommen. Auf diese Weise ist während eines Verschwenkens der Gelenkschenkel relativ zueinander ein Abstand zwischen dem zweiten Führungskörper und dem Kopplungskörper mittels des Kopplungselements vordefiniert und konstant. Dies begünstigt die Stabilität der Gelenkanordnung bei einer Verschwenkung der Gelenkschenkel relativ zueinander aufgrund der bestehenden drei Kontaktpunkte. Diese drei Kontaktpunkte bestehen zwischen der ersten Führungsausnehmung und dem ersten Führungskörper, zwischen der zweiten Führungsausnehmung und dem zweiten Führungskörper und zwischen der Kopplungsausnehmung und dem Kopplungskörper. Ferner begünstigt das Kopplungselement die relative Bewegung der Gelenkschenkel zueinander.

Vorzugsweise umfasst die Gelenkanordnung ein weiteres Kopplungselement. Weiter bevorzugt ist eines der Kopplungselemente benachbart des ersten Kopfabschnitts und weiter bevorzugt ist das weitere Kopplungselement benachbart des zweiten Kopfabschnitts derart angeordnet, dass die Kopplungselemente den ersten und zweiten Kopfabschnitt zwischen sich einschließen. Auf diese Weise wird die Stabilität der Gelenkanordnung weiter verbessert.

Ferner kann erfindungsgemäß vorgesehen sein, dass der zweite Kopfabschnitt eine bogenförmige dritte Führungsausnehmung aufweist, die den Kopplungskörper derart aufnimmt, dass eine relative Bewegung der bogenförmigen dritten Führungsausnehmung in Bezug auf den Kopplungskörper möglich ist. Die bogenförmige dritte Führungsausnehmung ermöglicht es, den zweiten Kopfabschnitt möglichst stabil und großflächig auszubilden, auch wenn die Gelenkanordnung zwei Kopplungselemente umfasst.

Gemäß einer Weiterbildung der vorliegenden Erfindung kann vorgesehen sein, dass die gekrümmte erste Führungsausnehmung zwei Innenseitenwände aufweist, zwischen denen der erste Führungskörper spielarm oder spielfrei aufgenommen ist, sodass eine relative Bewegung zwischen der ersten Führungsausnehmung und dem ersten Führungskörper auf die Längserstreckung der ersten Führungsausnehmung beschränkt ist. Zusätzliche oder alternativ kann die zweite Führungsausnehmung zwei Innenseitenwände aufweisen, zwischen denen der zweite Führungskörper spielarm oder spielfrei aufgenommen ist, sodass eine relative Bewegung zwischen der zweiten Führungsausnehmung und dem zweiten Führungskörper auf die Längserstreckung der zweiten Führungsausnehmung beschränkt ist. Alternativ kann die zweite Führungsausnehmung eine von der zweiten Bewegungsbahn des zweiten Führungskörpers abweichende Innenkontur aufweisen, solange die zweite Führungsausnehmung zwei Endanschläge bereitstellt, die die Bewegungsbahn des zweiten Führungskörpers begrenzen.

Im Rahmen der Beschreibung der vorliegenden Erfindung bedeutet der Begriff "spielfrei", dass ein geringfügiges Spiel aufgrund von Fertigungstoleranzen, wie beispielsweise zwischen 0,02 mm und 0,05 mm, vorhanden sein kann. Als "spielarm" wird in dieser Offenbarung ein geringfügiges Spiel verstanden, wie beispielsweise zwischen 0,05 mm und 0,1 mm. Eine spielarme oder spielfreie Aufnahme der Führungskörper in den zugeordneten Führungsausnehmungen ermöglicht eine reproduzierbare Verschwenkbewegung der Gelenkschenkel relativ zueinander.

Eine bevorzugte Ausführungsform der Erfindung sieht vor, dass in einer vollständig gestreckten Stellung der Gelenkschenkel zueinander der erste Führungskörper an einem in der ersten Führungsausnehmung angeordneten Streckungsanschlag anschlägt, und der zweite Führungskörper an einer in der zweiten Führungsausnehmung angeordneten Streckungsposition positioniert ist. Mit anderen Worten, der zweite Führungskörper schlägt vorzugsweise in der Streckungsposition an den Endanschlag der zweiten Führungsausnehmung an. Vorzugsweise befindet sich die Streckungsposition, also ein Endanschlag der zweiten Führungsausnehmung, an einem ersten Ende der zweiten Führungsausnehmung, das weiter entfernt von dem ersten Schenkelabschnitt vorgesehen ist. Der Streckungsanschlag definiert reproduzierbar die vollständig gestreckte Stellung der Gelenkschenkel zueinander. Ferner wird ein Überstrecken der Gelenkanordnung und folglich ein Überstrecken des Extremitätengelenks durch den Streckungsanschlag vermieden. Der zweite Führungskörper in der Streckungsposition, also an dem Endanschlag der zweiten Führungsausnehmung, kann die Drehachse der Gelenkschenkel definieren, wenn diese ausgehend von der vollständig gestreckten Position relativ zueinander verschwenkt werden. Bevorzugt sind in der vollständig gestreckten Stellung der Gelenkschenkel zueinander die Längsachsen beider Gelenkschenkel einander parallel oder weiter bevorzugt zueinander ausgerichtet.

Ferner sieht eine bevorzugte Ausführungsform der Erfindung vor, dass in einer vollständig gebeugten Stellung der Gelenkschenkel zueinander der erste Führungskörper an einem in der ersten Führungsausnehmung angeordneten Beugungsanschlag anschlägt und der zweite Führungskörper an einer in der zweiten Führungsausnehmung angeordneten Beugungsposition positioniert ist. Mit anderen Worten, der zweite Führungskörper schlägt vorzugsweise in der Beugungsposition an den anderen Endanschlag der zweiten Führungsausnehmung an. Vorzugsweise befindet sich die Beugungsposition, also der andere Endanschlag der zweiten Führungsausnehmung, an einem zweiten Ende der zweiten Führungsausnehmung, das näher an dem ersten Schenkelabschnitt vorgesehen ist. Der Beugungsanschlag definiert reproduzierbar die vollständig gebeugte Stellung der Gelenkschenkel zueinander. Eine Beugung über den gewünschten Beugewinkel hinaus wird folglich durch den Beugungsanschlag vermieden. Werden die Gelenkschenkel ausgehend von der vollständig gebeugten Position aufeinander zu bewegt, also in Richtung einer Streckbewegung bewegt, verläuft die Drehachse vorzugsweise senkrecht zur Schwenkebene und durch den zweiten Führungskörper, insbesondere durch dessen Längsachse.

Gemäß einer weiteren optionalen erfindungsgemäßen Ausgestaltung kann die gekrümmte erste Führungsausnehmung an wenigstens einem ihrer Längsenden eine Auskerbung aufweisen, in die ein Anschlagelement eingreift, das den Streckungsanschlag bzw. den Beugungsanschlag bildet. Das Anschlagelement kann aus einem anderem Material als das erste Kopfelement sein, um beispielsweise ein Anschlaggeräusch des ersten Führungskörpers zu dämpfen. Das Anschlagelement kann aus Kunststoff hergestellt sein. Die Gelenkschenkel können aus Aluminium oder einer Legierung davon hergestellt sein.

Das Anschlagelement kann austauschbar sein. Ein Teil des Anschlagelements kann derart in die gekrümmte erste Führungsausnehmung eingreifen, dass die relative Bewegung zwischen dem ersten Führungskörper und der ersten Führungsausnehmung durch diesen Teil des Anschlagelements bestimmt wird. Vorteilhafterweise können Anschlagelemente zusammen mit der Gelenkanordnung verwendet werden, bei denen der Teil des Anschlagelements, der in die erste Führungsausnehmung eingreift, unterschiedlich ausgebildet ist. Dies ermöglicht eine Einstellung der minimalen und maximalen Beugewinkel, wie später noch im Detail erläutert werden wird. Ferner kann die Gelenkanordnung individuell für jeden Patienten an unterschiedliche Therapieformen angepasst werden.

Eine bevorzugte Ausführungsform der Erfindung sieht vor, dass bei einer Beugebewegung der Gelenkschenkel aus einer vollständig gestreckten Stellung der Gelenkschenkel zueinander innerhalb eines ersten Beugungsbereichs eine Relativbewegung der ersten Führungsausnehmung und des darin aufgenommenen ersten Führungskörpers erfolgt, während die relative Lage der zweiten Führungsausnehmung und des darin aufgenommenen zweiten Führungskörpers unverändert bleibt, sodass der in der Streckungsposition positionierte zweite Führungskörper die Drehachse bildet. Der erste Beugungsbereich ermöglicht somit eine Drehbewegung der Gelenkschenkel in der Schwenkebene um eine stationäre Drehachse, die vorzugsweise senkrecht zu der Schwenkebene und durch den zweiten Führungskörper verläuft. Folglich kann ein Nachbilden des natürlichen physiologischen Bewegungsablaufs verbessert werden, wie zum Beispiel die Gleitbewegung bezogen auf den Oberschenkelknochen (Femur) und den Unterschenkelknochen (Tibia).

Weiterhin sieht eine bevorzugte Ausführungsform der Erfindung vor, dass bei einer Streckbewegung der Gelenkschenkel aus einer vollständig gebeugten Stellung der Gelenkschenkel zueinander innerhalb eines zweiten Beugungsbereichs eine Relativbewegung der ersten Führungsausnehmung und des darin aufgenommenen ersten Führungskörpers erfolgt, während die relative Lage der zweiten Führungsausnehmung und des darin aufgenommenen zweiten Führungskörpers unverändert bleibt, sodass der in der Beugungsposition positionierte zweite Führungskörper die Drehachse bildet. Der zweite Beugungsbereich ermöglicht somit eine Drehbewegung der Gelenkschenkel in der Schwenkebene um eine stationäre Drehachse, die vorzugsweise senkrecht zu der Schwenkebene und durch den zweiten Führungskörper verläuft. Folglich kann ein Nachbilden des natürlichen physiologischen Bewegungsablaufs verbessert werden.

Ferner sieht eine bevorzugte Ausführungsform der Erfindung vor, dass bei einer Beugebewegung innerhalb eines dritten Beugungsbereichs, der sich an den ersten oder/und den zweiten Beugungsbereich anschließt, sowohl eine Relativbewegung der ersten Führungsausnehmung und des darin aufgenommenen ersten Führungskörpers als auch eine Relativbewegung der zweiten Führungsausnehmung und des darin aufgenommenen zweiten Führungskörpers erfolgt, sodass der zweite Führungskörper eine sich verändernde Drehachse bildet, die in einem Bereich zwischen der Beugungsposition und der Streckungsposition veränderbar positioniert ist. Der dritte Beugungsbereich ermöglicht somit eine Rollbewegung der Gelenkschenkel in der Schwenkebene um die sich verändernde Drehachse. Folglich kann ein Nachbilden des natürlichen Bewegungsablaufs verbessert werden.

Gemäß einer weiteren optionalen erfindungsgemäßen Ausgestaltung kann der erste Beugungsbereich einen Winkelbereich umfassen, der zwischen der vollständig gestreckten Stellung der Gelenkschenkel zueinander und einem dazu verschwenkten Beugewinkel von zwischen 15° und 25°, insbesondere bei etwa 20°, liegt. Alternativ oder zusätzlich kann der zweite Beugungsbereich einen Winkelbereich umfassen, der zwischen einer vollständig gebeugten Stellung der Gelenkschenkel zueinander und einem Beugewinkel von zwischen 110° und 130°, insbesondere bei etwa 120°, liegt. Der Beugewinkel in der vollständig gebeugten Stellung beträgt vorzugsweise zwischen 135° und 145°, insbesondere etwa 140°. Somit kann mittels des zweiten Beugungsbereichs eine starke Beugung des Extremitätengelenks unterstützt werden. Alternativ oder zusätzlich kann der dritte Beugungsbereich einen Winkelbereich umfassen, der bei einem Beugewinkel von zwischen 15° und 25°, insbesondere von etwa 20°, beginnt und bei einem Beugewinkel zwischen 110° und 130°, insbesondere von etwa 120°, endet. Die Beugungsbereiche sind an den natürlichen Bewegungsablauf eines Extremitätengelenks angepasst und bilden diesen besonders gut ab.

Gemäß einer bevorzugten Ausführungsform der Erfindung kann vorgesehen sein, dass zwischen dem ersten und zweiten Kopfabschnitt ein reibungsminderndes Element, insbesondere eine Gleitscheibe, vorgesehen ist. Dadurch kann Reibung zwischen den Kopfabschnitten reduziert werden, wenn diese sich relativ zueinander bewegen und dabei miteinander kontaktieren. Insgesamt kann damit eine verbesserte Gelenkanordnung bereitgestellt werden, die leicht bewegbar ist.

Gemäß einem Aspekt der Erfindung kann vorgesehen sein, dass zumindest ein Verbindungsmittel, insbesondere ein Bolzen, eine Schraube oder ein Niet, und/oder ein formloser Hilfsstoff, bevorzugt ein Klebstoff, den ersten Führungskörper an dem zweiten Kopfabschnitt befestigt. Damit kann erreicht werden, dass der Führungskörper fest an dem zweiten Kopfabschnitt angebracht ist. Ferner kann der Führungskörper unabhängig von dem zweiten Kopfabschnitt gefertigt und anschließend können diese Komponenten miteinander montiert werden. Außerdem kann erreicht werden, dass der Führungskörper einfach an dem zweiten Kopfabschnitt angebracht werden kann.

Gemäß einem Aspekt der Erfindung kann die Gelenkanordnung ferner eine Polsterhalterung umfassen, die an dem Kopplungselement, das dem zweiten Kopfabschnitt benachbart ist, derart befestigt ist, dass sich das Kopplungselement und die Polsterhalterung simultan bewegen. Die Polsterhalterung kann somit die Gelenkanordnung abdecken und vor äußeren Einflüssen schützen. Die Polsterhalterung liegt vorzugsweise an einer Seite des Extremitätengelenks an. Somit kann die Polsterhalterung vorteilhafterweise einen Formschluss der Orthese zum Kniegelenk gewährleisten. Ferner kann die Polsterhalterung als seitliche Führung der Orthese am Kniegelenk dienen. Auch kann die Polsterhalterung ein unangenehmes Reiben der Gelenkanordnung an der Haut oder der Kleidung der Person verhindern, die die Gelenkanordnung, beispielsweise als Teil einer Orthese, trägt. Durch die simultane Bewegung der Polsterhalterung wird der Tragekomfort der Gelenkanordnung verbessert.

Eine bevorzugte Ausführungsform der Erfindung sieht vor, dass die Gelenkanordnung ferner eine Abdeckung umfasst, die zumindest einen Teil des ersten Kopfabschnitts bedeckt und, vorzugsweise mittels Rast-Ausnehmungen in entsprechende Aussparungen, in bzw. an dem ersten Kopfabschnitt abnehmbar befestigbar ist. Die Abdeckung kann vorteilhafterweise die Gelenkanordnung vor äußeren Einwirkungen, wie Schmutz oder leichten Schlägen, schützen.

Eine Weiterbildung der Erfindung sieht vor, dass das Extremitätengelenk ein Kniegelenk ist. Somit kann eine Gelenkanordnung bereitgestellt werden, die zum Tragen an einem Kniegelenk angepasst ist.

Die oben genannte Aufgabe wird ferner durch ein Set zum Konfigurieren der Gelenkanordnung gelöst, das zumindest eine Gelenkanordnung, wie zuvor beschrieben, sowie eine Mehrzahl von Anschlagelementen umfasst, die sich in der Längserstreckung des Teils unterscheiden, der in die gekrümmte erste Führungsausnehmung eingreift. Die Anschlagelemente können den ersten, zweiten oder/und dritten Beugungsbereich der Gelenkanordnung verringern. Zum Beispiel kann ein Anschlagelement vorgesehen sein, dessen in die gekrümmte erste Führungsausnehmung eingreifender Teil eine vollständige Streckung der Gelenkschenkel zueinander verhindert. Der erste Beugungsbereich kann dann einen Winkelbereich umfassen, der bei einem Beugewinkel von beispielsweise 5° beginnt und bei einem Beugewinkel zwischen 15° und 25°, vorzugsweise bei etwa 20°, endet. Ferner kann beispielsweise ein Anschlagelement vorgesehen sein, dessen in die gekrümmte erste Führungsausnehmung eingreifender Teil eine vollständige Beugung der Gelenkschenkel zueinander verhindert. Der zweite Beugungsbereich kann dann einen Winkelbereich umfassen, der bei einem Beugewinkel zwischen 110° und 130°, vorzugsweise etwa 120°, beginnt und bei einem Beugewinkel von etwa 135° endet. Auf diese Weise kann eine individuelle Anpassung der Gelenkanordnung an die gewünschte Therapieform erfolgen. Es kann erfindungsgemäß vorgesehen sein, dass die Anschlagelemente austauschbar sind und an den jeweiligen Therapieerfolg bedarfsweise angepasst werden können.

Das Set zum Konfigurieren der Gelenkanordnung gemäß der vorliegenden Erfindung kann erste Anschlagelemente umfassen, die den Beugungsbereich beginnend von der vollständig gestreckten Stellung der Gelenkschenkel zueinander um einen Beugewinkel von beispielsweise 5°, 10°, 15°, 20° oder 25° verkleinern. Alternativ oder zusätzlich kann das Set zum Konfigurieren der Gelenkanordnung zweite Anschlagelemente umfassen, die eine vollständig gebeugte Stellung der Gelenkschenkel verhindern und einen maximalen Beugewinkel von beispielsweise 125°, 110°, 90°, 70° oder 60° zulassen. Die ersten Anschlagelemente können den Streckungsanschlag und die zweiten Anschlagelemente können den Beugungsanschlag bilden. Es können eines oder mehrere der ersten oder/und zweiten Anschlagelemente in dem Set zum Konfigurieren der Gelenkanordnung enthalten sein, von denen jedes den Beugewinkel um einen definierten Betrag in Bezug auf die vollständig gestreckte oder/und vollständig gebeugte Stellung der Gelenkschenkel zueinander verringert.

Ferner wird die eingangs bezeichnete und der Erfindung zugrundeliegende Aufgabe durch eine Orthese gelöst, wobei die Orthese zumindest eine Gelenkanordnung oder ein Set zum Konfigurieren der Gelenkanordnung der vorangehenden beschriebenen Art umfasst.

Gemäß einer Weiterbildung der Erfindung kann vorgesehen sein, dass an der Orthese die zumindest eine Gelenkanordnung an einer lateralen oder einer medialen Seite des Extremitätengelenks angeordnet ist.

Die Erfindung wird im Folgenden beispielhaft anhand der beiliegenden Figuren erläutert. Es stellen dar:
- Fig. 1a-b: eine schematische Vorderansicht und eine schematische Rückansicht auf eine erfindungsgemäße Gelenkanordnung in einer ersten Stellung;
- Fig. 2a-b: schematische Draufsichten eines erfindungsgemäßen ersten Gelenkschenkels und eines erfindungsgemäßen zweiten Gelenkschenkels;
- Fig. 3a: eine schematische perspektivische Ansicht eines erfindungsgemäßen Gelenkschenkels mit einem ersten Führungskörper;
- Fig. 3b: eine schematische perspektivische Ansicht des ersten Führungskörpers von Fig. 3a;
- Fig. 4: eine schematische Seitenansicht einer erfindungsgemäßen Gelenkanordnung in der ersten Stellung;
- Fig. 5a-b: schematische perspektivische Ansichten einer erfindungsgemäßen Gelenkanordnung in einer zweiten Stellung;
- Fig. 6: eine schematische perspektivische Ansicht einer erfindungsgemäßen Gelenkanordnung in einer dritten Stellung;
- Fig. 7a-b: schematische perspektivische Ansichten einer Abdeckung der erfindungsgemäßen Gelenkanordnung; und
- Fig. 8a-d: verschiedene Anschlagelemente für ein erfindungsgemäßes Set zum Konfigurieren der Gelenkanordnung.

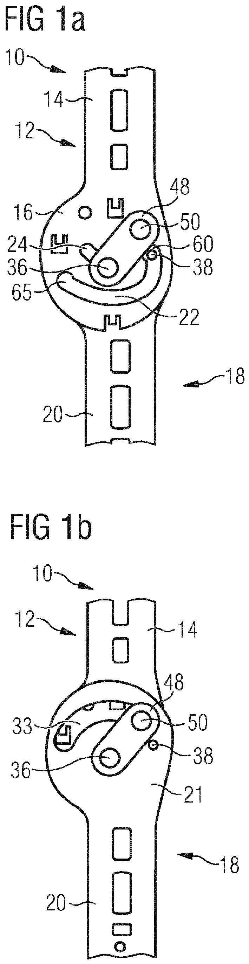

Die Figuren 1a-b zeigen schematische perspektivische Ansichten einer erfindungsgemäßen Gelenkanordnung 10 in einer ersten Stellung. In der ersten Stellung befindet sich die Gelenkanordnung 10 in einer vollständig gestreckten Stellung. Die Gelenkanordnung 10 umfasst einen ersten Gelenkschenkel 12 mit einem länglichen ersten Schenkelabschnitt 14 und einem ersten Kopfabschnitt 16. Ferner umfasst die Gelenkanordnung 10 einen zweiten Gelenkschenkel 18 mit einem länglichen zweiten Schenkelabschnitt 20 und einem zweiten Kopfabschnitt 21. Im Bereich des ersten und zweiten Kopfabschnitts 16, 21 überlappen sich der erste und zweite Gelenkschenkel 12, 18. Außerdem liegt der erste Kopfabschnitt 16 an dem zweiten Kopfabschnitt 21 an. Der längliche erste Schenkelabschnitt 14 und der längliche zweite Schenkelabschnitt 20 sind mit einer geschwungenen Linie jeweils verkürzt dargestellt. Dies soll verdeutlichen, dass die Länge des länglichen ersten und zweiten Schenkelabschnitts 14, 20 einsatzabhängig und/oder patientenabhängig gewählt werden kann. Ferner können nicht dargestellte Komponenten vorgesehen sein, um die Gelenkanordnung 10 in eine Orthese zu integrieren.

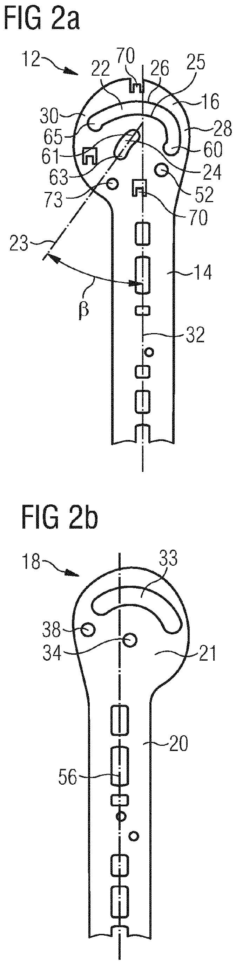

Der erste Gelenkschenkel 12 ist in Figur 2a in einer Seitenansicht losgelöst von anderen Komponenten der Gelenkanordnung dargestellt. Der erste Kopfabschnitt 16 umfasst eine gekrümmte erste Führungsausnehmung 22 sowie eine zweite Führungsausnehmung 24. Die gekrümmte erste Führungsausnehmung 22 ist in einem äußeren Endbereich des ersten Kopfabschnitts 16 angeordnet. Die gekrümmte erste Führungsausnehmung 22 definiert eine erste Bewegungsbahn zwischen ihren Innenseitenflächen mittig entlang ihrer Längserstreckung. Die erste Bewegungsbahn weist unterschiedliche Krümmungsradien auf, vorzugsweise mindestens drei unterschiedliche Krümmungsradien. Die gekrümmte erste Führungsausnehmung 22 erstreckt sich in der Seitenansicht von Figur 2a von einem ersten Seitenbereich 28 zu einem zweiten Seitenbereich 30 des ersten Kopfabschnitts 16. Bei einer Aufteilung des ersten Kopfabschnitts 16 in zwei gleichgroße Abschnitte kann die gekrümmte erste Führungsausnehmung 22 in lediglich einem der beiden Abschnitte vollständig angeordnet sein.

Eine Umfangsfläche der ersten Führungsausnehmung 22 weist einen im Wesentlichen konkaven Flächenabschnitt 25 und einen im Wesentlichen konvexen Flächenabschnitt 26 auf. Der konkave Flächenabschnitt 25 ist dem ersten Schenkelabschnitt 14 zugewandt und der konvexe Flächenabschnitt 26 ist dem ersten Schenkelabschnitt 14 abgewandt. Die Begriffe "konkav" und "konvex" beziehen sich hierin auf die Außenkontur der ersten Führungsausnehmung 22. Die konkaven und konvexen Flächenabschnitte 25, 26 sind in einem mittleren Bereich in Bezug auf die Längserstreckung der ersten Führungsausnehmung 22 angeordnet.

Die zweite Führungsausnehmung 24 des ersten Kopfabschnitts 16 ist in einem in etwa zentralen Bereich des ersten Kopfabschnitts 16 angeordnet. Unter einer zweiten Führungsausnehmung wird in dieser Offenbarung eine Ausnehmung verstanden, die zwei Endanschläge an ihren zwei Enden 61, 63, vorzugsweise Längsenden 61, 63, definiert. Ferner kann darunter eine längliche zweite Führungsausnehmung verstanden werden, die linear oder leicht gekrümmt sein kann, beispielsweise eine längliche Bohnenform aufweist. In der in Figur 2a dargestellten Ausführungsform ist die längliche zweite Führungsausnehmung 24 leicht gebogen. Die Biegung der zweiten Führungsausnehmung kann beispielsweise einen Radius mit einem Mittelpunkt aufweisen, der durch eine Achse des später eingeführten Kopplungskörpers 50 gebildet wird.

Eine Längsachse 23 der zweiten Führungsausnehmung 24 erstreckt sich unter einem Winkel β zu einer von dem ersten Schenkelabschnitt 14 definierten Längsachse 32, sodass sich die Längsachsen 23, 32 schneiden. Die Längsachse 23 verläuft durch die zwei Enden 61, 63 der zweiten Führungsausnehmung 24. Der Winkel β ist vorzugsweise zwischen 5° und 85°, besonders bevorzugt zwischen 70° und 30°. Die zweite Führungsausnehmung 24 definiert eine zweite Bewegungsbahn zwischen ihren Innenseitenflächen mittig entlang ihrer Längserstreckung.

Im Vergleich zu der Längserstreckung der zweiten Führungsausnehmung 24 ist die Längserstreckung der ersten Führungsausnehmung 22 wesentlich länger, beispielsweise mehr als doppelt so lang, weiter bevorzugt mehr als dreimal so lang. Die gekrümmte erste Führungsausnehmung 22 und die zweite Führungsausnehmung 24 sind voneinander beabstandet. Quererstreckungen der ersten Führungsausnehmung 22 und der zweiten Führungsausnehmung 24 können einander entsprechen oder sich geringfügig voneinander unterscheiden.

Der zweite Gelenkschenkel 18 ist in Figur 2b in einer Seitenansicht losgelöst von anderen Komponenten der Gelenkanordnung 10 dargestellt. Der zweite Kopfabschnitt 21 des zweiten Gelenkschenkels 18 umfasst eine bogenförmige dritte Führungsausnehmung 33 und eine zentrale Aussparung 34. In einem montierten Zustand der Gelenkanordnung, wie z.B. in Figuren 1a-b dargestellt, fällt die zentrale Aussparung 34 mit der zweiten Führungsausnehmung 24 des ersten Kopfabschnitts 16 zusammen, sodass ein zweiter Führungskörper 36 sowohl die zweite Führungsausnehmung 24 als auch die zentrale Aussparung 34 durchtritt. Der zweite Kopfabschnitt 21 ist ferner in einem seitlichen Bereich davon mit einem erster Führungskörper 38 gekoppelt. Der erste Führungskörper 38 steht von dem zweiten Kopfabschnitt 21 derart hervor, dass dieser in dem montierten Zustand der Gelenkanordnung 10 in die gekrümmte erste Führungsausnehmung 22 des ersten Kopfabschnitts 16 eingreift, wie in Figur 1a gezeigt.

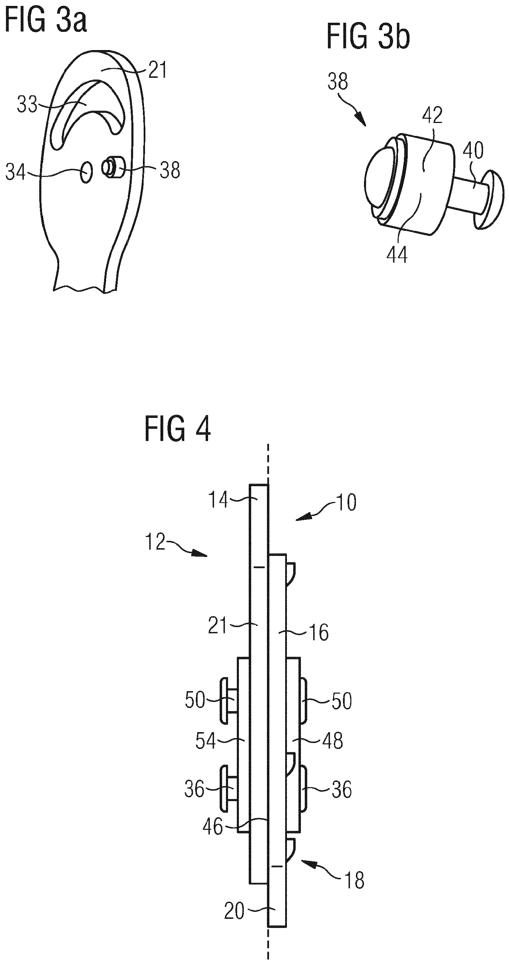

Figur 3a zeigt den zweiten Kopfabschnitt 21 in einer perspektivischen Ansicht mit dem davon hervorstehenden ersten Führungskörper 38. Eine perspektivische Ansicht des ersten Führungskörpers 38 ist in Figur 3b dargestellt. Der erste Führungskörper 38 kann ein Niet, ein Bolzen, eine Schraube oder dergleichen sein. In der dargestellten Ausführungsform ist der erste Führungskörper 38 ein Bolzen, der einen Befestigungsabschnitt 40 zur Befestigung an dem zweiten Kopfabschnitt 21 und einen Führungsabschnitt 42 zum Eingreifen in die gekrümmte erste Führungsausnehmung 22 umfasst. Der Führungsabschnitt 42 des ersten Führungskörpers 38 kann eine reibungsmindernde Schicht 44, wie beispielsweise eine Lage aus Teflon (PTFE), an seiner Oberfläche umfassen. Die reibungsmindernde Schicht 44 liegt spielarm oder spielfrei an den Innenumfangsflächen 25, 26 der ersten Führungsausnehmung 22 an, wie in Figur 1a gezeigt.

Eine Befestigung der beiden Gelenkschenkel 12, 18, genauer gesagt der beiden Kopfabschnitte 16, 21 davon, aneinander erfolgt mittels eines Kopplungselements 48, das in Figur 1a in einer perspektivischen und in Figur 4 in einer Seitenansicht dargestellt ist. Das Kopplungselement 48 koppelt die beiden Kopfabschnitte 16, 21 miteinander. Es hält ferner die beiden Kopfabschnitte in einem definierten Abstand zueinander, sodass eine Verschwenkung der Gelenkschenkel 12, 18 relativ zueinander in einer Schwenkebene 46 möglich ist. Das Kopplungselement 48 ist mit dem zweiten Führungskörper 36 und einem Kopplungskörper 50 gekoppelt, der sich in eine Kopplungsaussparung 52 des ersten Kopfabschnitts 16 erstreckt. Die Kopplungsaussparung 52 nimmt den Kopplungskörper 50 spielarm oder im Wesentlichen spielfrei derart auf, dass der Kopplungskörper 50 relativ zu der Kopplungsaussparung 52 ortsfest ist. Der Kopplungskörper 50 ist ortsfest und drehbar in der Kopplungsaussparung 52 aufgenommen. Der Kopplungskörper 50 durchtritt ferner die bogenförmige dritte Führungsausnehmung 33 des zweiten Kopfabschnitts 21. Die bogenförmige dritte Führungsausnehmung 33 ermöglicht eine relative Bewegung zwischen dem zweiten Kopfabschnitt 21 und dem Kopplungskörper 50. Der zweite Führungskörper 36 oder/und der Kopplungskörper 50 können ein Bolzen, eine Schraube oder ein Niet sein.

Die Gelenkanordnung 10 umfasst ein weiteres Kopplungselement 54. Wie in den Figuren 1a-b und in Figur 4 gezeigt, ist das Kopplungselement 48 benachbart des ersten Kopfabschnitts 16 angeordnet und ist das weitere Kopplungselement 54 benachbart des zweiten Kopfabschnitts 21 angeordnet. Die Kopplungselemente 48, 54 schließen den ersten und den zweiten Kopfabschnitt 16, 21 zwischen sich ein.

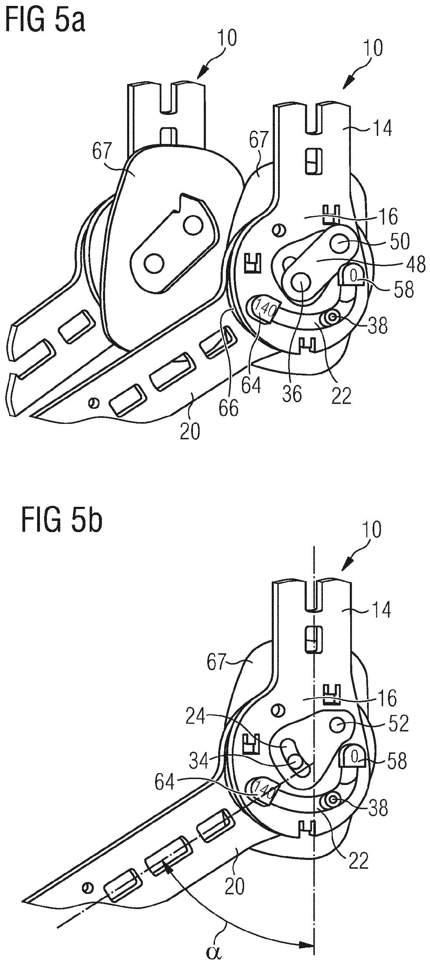

Im Folgenden wird ein Verschwenken der Gelenkschenkel 12, 18 relativ zueinander in der Schwenkebene 46 beschrieben. In der in Figuren 1a-b dargestellten vollständig gestreckten Stellung der Gelenkschenkel 12, 18 zueinander sind die Längsachsen 32, 56 des ersten bzw. des zweiten Schenkelabschnitts 14, 20 parallel zueinander ausgerichtet. Der erste Führungskörper 38 schlägt an einem Streckungsanschlag 59 an, der in Figur 1a der Übersichtlichkeit halber weggelassen ist. Der Streckungsanschlag 59 wird von einem ersten Anschlagelement 58 gebildet, wie in den Figuren 5a-b gezeigt. Das erste Anschlagelement 58 greift formschlüssig in eine Auskerbung 60 an einem Längsende der ersten Führungsausnehmung 22 ein. Ferner liegt ein Abschnitt des Anschlagelements 58 auf einer Oberfläche des ersten Kopfabschnitts 16 auf. Beides, das formschlüssige Eingreifen des Anschlagelements 58 in die Auskerbung 60 und die Auflage 82 des Anschlagelements 58 auf der Oberfläche des ersten Kopfabschnitts 16, ermöglichen eine definierte Positionierung des Anschlagelements 58 in Bezug auf die gekrümmte erste Führungsausnehmung 22.

In der Streckungsposition befindet sich der zweite Führungskörper 36 an dem ersten Ende 61 der zweiten Führungsausnehmung 24, das sich näher an der ersten Führungsausnehmung 22 als an dem Schenkelabschnitt 14 befindet. Zum Beispiel schlägt der zweite Führungskörper 36 in der Streckungsposition an das erste Ende 61 an. Das erste Ende 61 definiert somit einen der beiden Endanschläge der zweiten Führungsausnehmung 24. Werden die Gelenkschenkel 12, 18 relativ zueinander bewegt, bewegen sich die Schenkelabschnitte 14, 20 aufeinander zu und die Längsachsen 32, 56 der Schenkelabschnitte 14, 20 schließen einen Beugewinkel α zwischen sich ein. Während des Verschwenkens der Gelenkschenkel 12, 18 relativ zueinander, bewegt sich der erste Führungskörper 38 entlang der ersten Bewegungsbahn. Die erste Bewegungsbahn verläuft entlang der Längserstreckung der ersten Führungsausnehmung und mittig zwischen Innenseitenflächen der ersten Führungsausnehmung 22. Innerhalb eines ersten Beugungsbereichs, der bei der vollständig gestreckten Stellung der Gelenkschenkel 12, 18 zueinander beginnt, und bei einem Beugewinkel zwischen 15° und 25°, insbesondere bei etwa 20°, endet, erfolgt eine Drehbewegung der Gelenkschenkel 12, 18 zueinander um eine im Wesentlichen feststehende unveränderliche Drehachse. Diese feststehende unveränderliche Drehachse verläuft senkrecht zur Schwenkebene 46 und durch den zweiten Führungskörper 36.

Werden die Gelenkschenkel 12, 18 über den ersten Beugungsbereich hinaus weiter relativ zueinander verschwenkt, geht die Drehbewegung in eine Rollbewegung mit sich verändernder Drehachse über. Diese sich verändernde Drehachse verläuft senkrecht zur Schwenkebene 46 und durch den zweiten Führungskörper 36, jedoch bewegt sich der zweite Führungskörper 36 während der Rollbewegung entlang der zweiten Bewegungsbahn. Bei einem fortschreitenden Verschwenken der Gelenkschenkel 12, 18 zueinander bewegt sich der zweite Führungskörper 36 von der Streckungsposition an dem ersten Ende 61 der zweiten Führungsausnehmung 24 in Richtung einer Beugungsposition. In der Beugungsposition befindet sich der zweite Führungskörper 36 an einem zweiten Ende 63 der zweiten Führungsausnehmung 24, das näher an dem Schenkelabschnitt 14 als an der ersten Führungsausnehmung 22 liegt. Zum Beispiel schlägt der zweite Führungskörper 36 in der Beugungsposition an das zweite Ende 63 an.

Während der Rollbewegung folgt der zweite Führungskörper 36 der zweiten Bewegungsbahn, während gleichzeitig der erste Führungskörper 38 der durch die gekrümmte erste Führungsausnehmung 22 definierten ersten Bewegungsbahn folgt. Die zweite Bewegungsbahn des zweiten Führungskörpers 36 ist durch das mit diesem verbundene Kopplungselement 48 definiert und durch die Endanschläge an den Enden 61, 63 der zweiten Führungsausnehmung 24 begrenzt. In der dargestellten Ausführungsform ist die Innenkontur der zweiten Führungsausnehmung 24 entsprechend der zweiten Bewegungsbahn definiert.

Die Figuren 5a-b zeigen eine zweite Stellung der Gelenkschenkel 12, 18 zueinander, bei der diese derart relativ zueinander verschwenkt sind, dass eine Rollbewegung vorliegt. In der zweiten Stellung befindet sich der erste Führungskörper 38 in einem mittleren Bereich entlang der Längserstreckung der ersten Führungsausnehmung 22 und es befindet sich der zweite Führungskörper 36 zwischen dem ersten und dem zweiten Ende 61, 63 der zweiten Führungsausnehmung 26. In Figur 5b ist das Kopplungselement 48, der zweite Führungskörper 36 sowie der Kopplungskörper 50 der Übersichtlichkeit halber weggelassen, wodurch die Position der zentralen Aussparung 34 in Bezug auf die zweite Führungsausnehmung 24 sichtbar ist. Der zweite Führungskörper 36, der sich durch die zweite Führungsausnehmung 24 und die zentrale Aussparung 34 erstreckt, ist also in einem mittleren Bereich entlang der Längserstreckung der zweiten Führungsausnehmung 24 angeordnet. Eine Rollbewegung liegt in einem Beugungsbereich vor, der bei einem Beugewinkel α zwischen 15° und 25°, vorzugsweise etwa 20°, beginnt und bei einem Beugewinkel α zwischen 110° und 130°, vorzugsweise etwa 120°, endet.

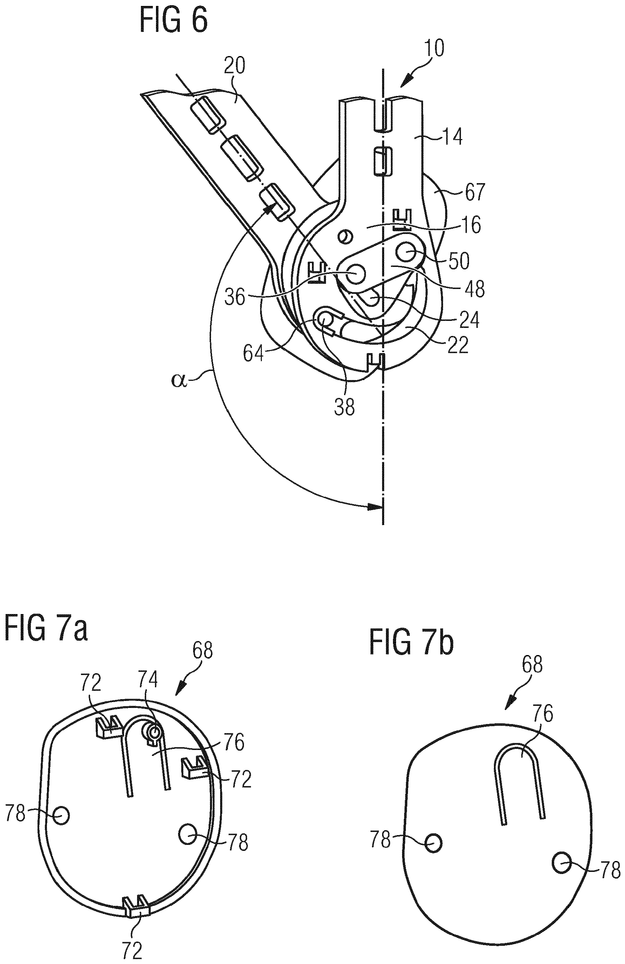

Die Gelenkschenkel 12, 18 können weiter relativ zueinander verschwenkt werden bis in eine dritte Stellung. In der dritten Stellung befindet sich die Gelenkanordnung 10 in einer vollständig gebeugten Stellung, wie in Figur 6 gezeigt. Der Beugewinkel α zwischen den Längsachsen 32, 56 der Schenkelabschnitte 14, 20 beträgt in der vollständig gebeugten Stellung zwischen 135° und 145°, insbesondere etwa 140°. In der vollständig gebeugten Stellung schlägt der erste Führungskörper 38 an einem Beugungsanschlag 62 an, der von einem zweiten Anschlagelement 64 gebildet wird. Das zweite Anschlagelement 64 ist in Figur 6 lediglich teilweise dargestellt, um dessen Innenkontur zu zeigen, an der der erste Führungskörper 38 anschlägt. Figur 6 zeigt denjenigen Abschnitt des zweiten Anschlagelements 64, der formschlüssig in eine Auskerbung 65 der ersten Führungsausnehmung 22 eingreift. Das zweite Anschlagelement 64 umfasst ferner eine Auflage 82, die auf einer Oberfläche des ersten Kopfabschnitts 16 aufliegt.

In der vollständig gebeugten Stellung ist der zweite Führungskörper 36 an dem zweiten Ende 63 der zweiten Führungsausnehmung 24 angeordnet. In einem zweiten Beugungsbereich, in dem der Beugewinkel α größer ist als 110°, vorzugsweise als 120°, weiter bevorzugt als 130°, bewegen sich die Gelenkschenkel 12, 18 in einer Gleitbewegung relativ zueinander. Die ortsfeste Drehachse verläuft senkrecht zur Schwenkebene 46 und durch den zweiten Führungskörper 36, der in der Beugungsposition an dem zweiten Ende 63 der zweiten Führungsausnehmung 24 angeordnet ist. Das zweite Ende 63 definiert somit den anderen der beiden Endanschläge der zweiten Führungsausnehmung 24.

Ferner umfasst die Gelenkanordnung 10 ein reibungsminderndes Element 66, das zwischen dem ersten Kopfabschnitt 16 und dem zweiten Kopfabschnitt 21 angeordnet und in Figur 5a zumindest ansatzweise sichtbar ist.

Die beispielhaft in den Figuren 5a-b und 6 dargestellte Gelenkanordnung 10 umfasst ferner eine Polsterhalterung 67. Die Polsterhalterung 67 ist mit dem Kopplungselement 54 gekoppelt, wie in Figur 5a ersichtlich. Folglich folgt die Polsterhalterung 67 einer Bewegung des Kopplungselements 54. Die Polsterhalterung 67 bewegt sich also immer dann, wenn die Gelenkschenkel 12, 18 einen Beugewinkel α relativ zueinander einnehmen, bei dem eine Rollbewegung der Gelenkschenkel 12, 18 zueinander vorliegt. Der dritte Beugungsbereich beginnt beispielsweise bei einem Beugewinkel α zwischen 15° und 25°, insbesondere bei etwa 20°, und endet beispielsweise bei einem Beugewinkel α zwischen 110° und 130°, insbesondere bei etwa 120°.

Figur 7 zeigt eine Abdeckung 68, die an dem ersten Kopfabschnitt 16 befestigbar ist, und zwar an der Seite des ersten Kopfabschnitts 16, die dem zweiten Kopfabschnitt 21 abgewandt ist. Zur Befestigung der Abdeckung 68 weist der erste Kopfabschnitt 16 Rast-Ausnehmungen 70 auf, in die Rastelemente 72 der Abdeckung 68 eingreifen können. Ferner weist der erste Kopfabschnitt 16 eine Rastaussparung 73 auf, in die ein weiteres Rastelement 74 der Abdeckung 68 eingreifen kann. Die Abdeckung 68 umfasst ferner eine Grifflasche 76. Zum Entfernen der Abdeckung 68 von dem ersten Kopfabschnitt 16 kann der Benutzer in die Grifflasche 76 der Abdeckung 68 greifen, das weitere Rastelement 74 aus der Rastaussparung 73 lösen und anschließend die Rastelemente 72 außer Eingriff mit den Rast-Ausnehmungen 70 bringen. Die Abdeckung 68 weist ferner zwei Sichtfenster 78 auf, die mit einer Oberfläche der Anschlagelemente 58, 64 fluchten. Die Oberfläche der Anschlagelemente 58, 64 kann eine Kennzeichnung des Beugewinkels α umfassen, den das Anschlagelement ermöglicht, wie beispielsweise die Kennzeichnung "0" für einen Beugewinkel α von 0° oder die Kennzeichnung "140" für einen Beugewinkel α von 140°, wie in Figuren 5a-b gezeigt. Ein Beugewinkel von 0° entspricht der vollständig gestreckten Stellung. Dabei zeigt die Kennzeichnung des ersten Anschlagelements 58 den minimalen und die Kennzeichnung des zweiten Anschlagelements 64 den maximalen Beugewinkel der Gelenkschenkel 12, 18 zueinander an.

Die Anschlagelemente 58, 64 sind lösbar mit dem ersten Kopfabschnitt 16 verbunden und austauschbar. Das erste Anschlagelement 58 kann sich mit einem Teil 80 davon in die erste Führungsausnehmung 22 erstrecken und einen Anschlag für den ersten Führungskörper 38 bilden, der eine Verschwenkung der Gelenkschenkel 12, 18 zueinander auf einen Beugungsbereich einschränkt, dessen minimaler Beugewinkel α beispielsweise 5°, 10°, 15°, 20° oder 25° beträgt. Auf gleiche Weise kann sich das zweite Anschlagelement 64 mit einem Teil 80 davon in die erste Führungsausnehmung 22 erstrecken und einen Anschlag 62 für den ersten Führungskörper 38 bilden, der eine Verschwenkung der Gelenkschenkel 12, 18 zueinander auf einen Beugungsbereich einschränkt, dessen maximaler Beugewinkel α beispielsweise 125°, 110°, 90°, 70° oder 60° beträgt. Es versteht sich, dass eine Mehrzahl verschiedener Anschlagelemente 58, 64 bereitgestellt werden kann, die mit dem ersten Kopfabschnitt 16 lösbar verbindbar sind. Jedes Anschlagelement 58, 64 kann unterschiedlich weit in die erste Führungsausnehmung 22 eingreifen, sodass jeder beliebige Beugungsbereich einstellbar ist. Der sich in die gekrümmte erste Führungsausnehmung 22 erstreckende Teil 80 des Anschlagelements 58, 64 weist eine Außenkontur auf, die der Innenkontur der ersten Führungsausnehmung 22 entspricht. An der Oberfläche jedes der Anschlagelemente 58, 64 kann der minimale bzw. maximale Beugewinkel α gekennzeichnet sein, sodass diese durch die Sichtfenster 78 in der Abdeckung 68 für den Benutzer sichtbar sind.

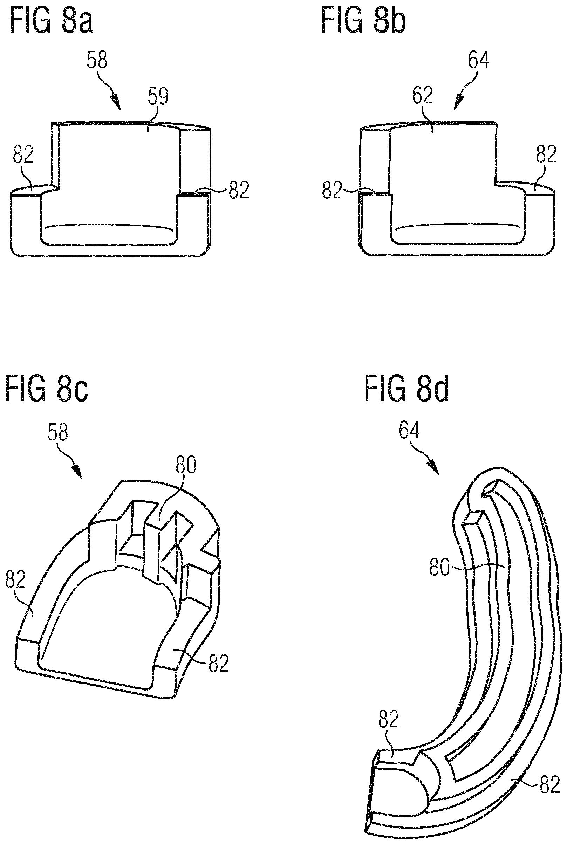

Figuren 8a-d zeigen beispielhafte Anschlagelemente 58, 64. Figur 8a zeigt das erste Anschlagelement 58 in einer Vorderansicht, das den Streckungsanschlag 59 aufweist, der eine vollständig gestreckte Stellung der Gelenkschenkel 12, 18 definiert. Figur 8b zeigt das zweite Anschlagelement 58 in einer Vorderansicht, das einen Beugungsanschlag 62 aufweist, der eine vollständig gebeugte Stellung der Gelenkschenkel 12, 18 definiert. Figur 8c zeigt das erste Anschlagelement 58 mit dem Teil 80, der sich in die erste Führungsausnehmung 22 erstreckt und einen minimalen Beugewinkel α zwischen den Gelenkschenkeln 12, 18 von 5° definiert. Figur 8d zeigt das zweite Anschlagelement 64 mit einem Teil 80, der sich in die erste Führungsausnehmung 22 erstreckt und einen maximalen Beugewinkel α zwischen den Gelenkschenkeln 12, 18 von 30° definiert. Ferner weisen die Anschlagelemente 58, 64 einen Abschnitt 82 auf, der dazu eingerichtet ist, auf einer Oberfläche des ersten Kopfabschnitts 16 aufzuliegen.

Die Gelenkanordnung 10 kann als Set zum Konfigurieren der Gelenkanordnung zusammen mit mehreren verschiedenen ersten und zweiten Anschlagelementen 58, 64 bereitgestellt sein.

Die Gelenkanordnung 10 kann Teil einer Orthese sein, beispielsweise einer Hartrahmenorthese. Eine Orthese kann beispielsweise zwei Gelenkanordnungen 10 umfassen, von denen eine benachbart der Innenseite und eine zweite benachbart der Außenseite des Extremitätengelenks angeordnet ist. Die Gelenkanordnungen 10 können in der Orthese spiegelsymmetrisch angeordnet sein, wie in Figur 5a dargestellt. Der zweite Kopfabschnitt 21 der Gelenkanordnung 10 kann dem Extremitätengelenk zugewandt sein. Die Orthese kann eine Kniegelenk-Orthese sein und die Kopfabschnitte 16, 21 der Gelenkanordnung 10 können dem Kniegelenk benachbart sein, während sich die Schenkelabschnitte 14, 20 der Gelenkanordnung 10 zumindest teilweise benachbart des Oberschenkelknochens (Femur) und des Unterschenkelknochens (Tibia) erstrecken.

Die in den Figuren dargestellten schematischen Ansichten können in Bezug auf das menschliche Extremitätengelenk medial oder lateral angeordnet sein. Die in den Figuren dargestellten schematischen Ansichten können daher je nach Anordnung der Gelenkanordnung spielverkehrt oder/und gedreht implementiert sein.

## Patentansprüche

1. Gelenkanordnung (10) für eine Orthese eines Extremitätengelenks, insbesondere zur Anbringung an einem Kniegelenk, umfassend:
- einen ersten Gelenkschenkel (12) mit einem länglichen ersten Schenkelabschnitt (14) und einem ersten Kopfabschnitt (16); und
- einen zweiten Gelenkschenkel (18) mit einem länglichen zweiten Schenkelabschnitt (20) und einem zweiten Kopfabschnitt (21);
wobei sich der erste Gelenkschenkel (12) und der zweite Gelenkschenkel (18) im Bereich des ersten und des zweiten Kopfabschnitts (16, 21) zumindest teilweise überlappen und ferner derart gelenkig miteinander verbunden sind, dass die Gelenkschenkel (12, 18) in einer Schwenkebene (46) zueinander verschwenkbar sind; und
wobei der erste Kopfabschnitt (16) umfasst:
- eine gekrümmte erste Führungsausnehmung (22), in die ein erster Führungskörper (38) derart eingreift, dass dieser relativ zu der ersten Führungsausnehmung (22) bewegbar ist, und
- eine von der ersten Führungsausnehmung (22) beabstandete zweite Führungsausnehmung (24), in die ein zweiter Führungskörper (36) derart eingreift, dass dieser relativ zu der zweiten Führungsausnehmung (24) bewegbar ist,
wobei der erste Führungskörper (38) mit dem zweiten Kopfabschnitt (21) gekoppelt ist, und wobei sich während einem Verschwenken der Gelenkschenkel (12, 18) zueinander
- der erste Führungskörper (38) entlang einer durch die Innenkontur der ersten Führungsausnehmung (22) definierten ersten Bewegungsbahn relativ zu der ersten Führungsausnehmung (22) bewegt, und
- der zweite Führungskörper (36) entlang einer zweiten Bewegungsbahn relativ zu der zweiten Führungsausnehmung (24) bewegt,
**dadurch gekennzeichnet, dass**
die gekrümmte erste Führungsausnehmung (22) eine Umfangsfläche aufweist, die einen im Wesentlichen konkaven und einen im Wesentlichen konvexen Flächenabschnitt (25, 26) aufweist, wobei der im Wesentlichen konkave Flächenabschnitt (25) der Umfangsfläche der ersten Führungsausnehmung (22) dem ersten Schenkelabschnitt (14) zugewandt ist und der im Wesentlichen konvexe Flächenabschnitt (26) der Umfangsfläche der ersten Führungsausnehmung (22) von dem ersten Schenkelabschnitt (14) abgewandt ist, und dass eine Längsachse (23) der zweiten Führungsausnehmung (24) eine von dem ersten Schenkelabschnitt (14) definierte Längsachse (32) unter einem Winkel (β) schneidet, der im Bereich zwischen 5° und 85° liegt.

2. Gelenkanordnung (10) nach Anspruch 1, **dadurch gekennzeichnet, dass** der erste Führungskörper (38) mit einem seitlichen Bereich des zweiten Kopfabschnitts (21) gekoppelt ist, wobei insbesondere der zweite Führungskörper (36) mit einem zentralen Bereich des zweiten Kopfabschnitts (21) gekoppelt ist, insbesondere diesen durchtritt.

3. Gelenkanordnung (10) nach Anspruch 1 oder 2, **dadurch gekennzeichnet, dass** die Gelenkanordnung (10) ferner ein Kopplungselement (48, 54) umfasst, mittels dem der erste Kopfabschnitt (16) und der zweite Kopfabschnitt (21) aneinander befestigt sind, wobei insbesondere das Kopplungselement (48, 54) mit dem zweiten Führungskörper (36) und einem Kopplungskörper (50) gekoppelt ist, der mit dem ersten Kopfabschnitt (16) gekoppelt ist.

4. Gelenkanordnung (10) nach Anspruch 3, **dadurch gekennzeichnet, dass** die Gelenkanordnung (10) ein weiteres Kopplungselement (54) umfasst, wobei eines der Kopplungselemente (48) benachbart des ersten Kopfabschnitts (16) und das weitere Kopplungselement (54) benachbart des zweiten Kopfabschnitts (21) derart angeordnet sind, dass die Kopplungselemente (48, 54) den ersten und zweiten Kopfabschnitt (16, 21) zwischen sich einschließen.

5. Gelenkanordnung (10) nach Anspruch 3 oder 4, **dadurch gekennzeichnet, dass** der zweite Kopfabschnitt (21) eine bogenförmige dritte Führungsausnehmung (33) aufweist, die den Kopplungskörper (50) derart aufnimmt, dass eine relative Bewegung der bogenförmigen Ausnehmung (33) in Bezug auf den Kopplungskörper (50) möglich ist.

6. Gelenkanordnung (10) nach einem der vorangehenden Ansprüche, **dadurch gekennzeichnet, dass**
- die gekrümmte erste Führungsausnehmung (22) zwei Innenseitenwände aufweist, zwischen denen der erste Führungskörper (38) spielarm oder spielfrei aufgenommen ist, sodass eine relative Bewegung zwischen der ersten Führungsausnehmung (22) und dem ersten Führungskörper (38) auf die Längserstreckung der ersten Führungsausnehmung (22) beschränkt ist,
oder/und
- die zweite Führungsausnehmung (24) zwei Innenseitenwände aufweist, zwischen denen der zweite Führungskörper (36) spielarm oder spielfrei aufgenommen ist, sodass eine relative Bewegung zwischen der zweiten Führungsausnehmung (24) und dem zweiten Führungskörper (36) auf die Längserstreckung der zweiten Führungsausnehmung (24) beschränkt ist.

7. Gelenkanordnung (10) nach einem der vorangehenden Ansprüche, **dadurch gekennzeichnet, dass**
in einer vollständig gestreckten Stellung der Gelenkschenkel (12, 18) zueinander
- der erste Führungskörper (38) an einem in der ersten Führungsausnehmung (22) angeordneten Streckungsanschlag (59) anschlägt, und
- der zweite Führungskörper (36) an einer in der zweiten Führungsausnehmung (24) angeordneten Streckungsposition positioniert ist, die sich insbesondere an einem ersten Ende (61) der zweiten Führungsausnehmung (24) befindet, das weiter entfernt von dem ersten Schenkelabschnitt (14) vorgesehen ist,
oder/und
in einer vollständig gebeugten Stellung der Gelenkschenkel (12, 18) zueinander
- der erste Führungskörper (38) an einem in der ersten Führungsausnehmung (22) angeordneten Beugungsanschlag (62) anschlägt, und
- der zweite Führungskörper (36) an einer in der zweiten Führungsausnehmung (24) angeordneten Beugungsposition positioniert ist, die sich insbesondere an einem zweiten Ende (63) der zweiten Führungsausnehmung (24) befindet, das näher an dem ersten Schenkelabschnitt (14) vorgesehen ist.

8. Gelenkanordnung (10) nach Anspruch 7, **dadurch gekennzeichnet, dass** die gekrümmte erste Führungsausnehmung (22) an wenigstens einem ihrer Längsenden eine Auskerbung (60, 65) aufweist, in die ein Anschlagelement (58, 64) eingreift, das den Streckungsanschlag (59) bzw. den Beugungsanschlag (62) bildet, wobei insbesondere das Anschlagelement (58, 64) austauschbar ist und ein Teil des Anschlagelements (80) derart in die gekrümmte erste Führungsausnehmung (22) eingreift, dass die relative Bewegung zwischen dem ersten Führungskörper (38) und der ersten Führungsausnehmung (22) durch diesen Teil des Anschlagelements (80) bestimmt wird.

9. Gelenkanordnung (10) nach Anspruch 7 oder 8, **dadurch gekennzeichnet, dass**
- bei einer Beugebewegung der Gelenkschenkel (12, 18) aus einer vollständig gestreckten Stellung der Gelenkschenkel (12, 18) zueinander innerhalb eines ersten Beugungsbereichs eine Relativbewegung der ersten Führungsausnehmung (22) und des darin aufgenommenen ersten Führungskörpers (38) erfolgt, während die relative Lage der zweiten Führungsausnehmung (24) und des darin aufgenommenen zweiten Führungskörpers (36) im Wesentlichen unverändert bleibt, sodass der in der Streckungsposition positionierte zweite Führungskörper (36) die Drehachse bildet, oder/und
- bei einer Streckbewegung der Gelenkschenkel (12, 18) aus einer vollständig gebeugten Stellung der Gelenkschenkel (12, 18) zueinander innerhalb eines zweiten Beugungsbereichs eine Relativbewegung der ersten Führungsausnehmung (22) und des darin aufgenommenen ersten Führungskörpers (38) erfolgt, während die relative Lage der zweiten Führungsausnehmung (24) und des darin aufgenommenen zweiten Führungskörpers (36) unverändert bleibt, sodass der in der Beugungsposition positionierte zweite Führungskörper (36) die Drehachse bildet,
oder/und
- bei einer Beugebewegung innerhalb eines dritten Beugungsbereichs, der sich an den ersten oder/und den zweiten Beugungsbereich anschließt, sowohl eine Relativbewegung der ersten Führungsausnehmung (22) und des darin aufgenommenen ersten Führungskörpers (38) als auch eine Relativbewegung der zweiten Führungsausnehmung (24) und des darin aufgenommenen zweiten Führungskörpers (36) erfolgt, sodass der zweite Führungskörper (36) eine sich verändernde Drehachse bildet, die in einem Bereich zwischen der Beugungsposition und der Streckungsposition veränderbar positioniert ist.

10. Gelenkanordnung (10) nach Anspruch 9, **dadurch gekennzeichnet, dass**
- der erste Beugungsbereich einen Winkelbereich umfasst, der zwischen der vollständig gebeugten Stellung der Gelenkschenkel (12, 18) zueinander und einem davon verschwenkten Beugewinkel (a) zwischen 15° und 25°, insbesondere bei etwa 20°, liegt, oder/und
- der zweite Beugungsbereich einen Winkelbereich umfasst, der zwischen der vollständig gebeugten Stellung der Gelenkschenkel (12, 18) zueinander und einem Beugewinkel (a) zwischen 110° und 130°, insbesondere bei etwa 120°, liegt, oder/und
- der dritte Beugungsbereich einen Winkelbereich umfasst, der bei einem Beugewinkel (a) zwischen 15° und 25°, insbesondere bei etwa 20°, beginnt und bei einem Beugewinkel (a) zwischen 110° und 130°, insbesondere bei etwa 120°, endet.

11. Gelenkanordnung (10) nach einem der vorangehenden Ansprüche, **dadurch gekennzeichnet, dass** zwischen dem ersten Kopfabschnitt (16) und dem zweiten Kopfabschnitt (21) ein reibungsminderndes Element (66), insbesondere eine Gleitscheibe, vorgesehen ist, wobei insbesondere der erste Führungskörper (38) an dem zweiten Kopfabschnitt (21) über zumindest ein Verbindungsmittel (40), insbesondere ein Bolzen, eine Schraube oder ein Niet, und/oder einen formlosen Hilfsstoff, bevorzugt ein Klebstoff, befestigt ist.

12. Gelenkanordnung (10) nach einem der Ansprüche 3 bis 11, **dadurch gekennzeichnet, dass** die Gelenkanordnung (10) ferner eine Polsterhalterung (67) umfasst, die an dem Kopplungselement (54), das dem zweiten Kopfabschnitt (21) benachbart ist, derart befestigt ist, dass sich das Kopplungselement (54) und die Polsterhalterung (67) simultan bewegen, wobei insbesondere die Gelenkanordnung (10) ferner eine Abdeckung (68) umfasst, die zumindest einen Teil des ersten Kopfabschnitts (16) bedeckt und in dem ersten Kopfabschnitt, vorzugsweise mittels Rast-Ausnehmungen (70) in entsprechende Aussparungen (72, 16) abnehmbar befestigbar ist.

13. Gelenkanordnung (10) nach einem der vorangehenden Ansprüche, **dadurch gekennzeichnet, dass** die zweite Führungsausnehmung näher an dem ersten Schenkelabschnitt angeordnet ist als die gekrümmte erste Führungsausnehmung (22).

14. Set zum Konfigurieren der Gelenkanordnung, umfassend zumindest eine Gelenkanordnung (10) nach einem der Ansprüche 1 bis 13 sowie eine Mehrzahl von Anschlagelementen (58, 64), die sich in der Längserstreckung des Teils (80) unterscheiden, der in die gekrümmte erste Führungsausnehmung (22) eingreift.

15. Orthese, umfassend zumindest eine Gelenkanordnung (10) nach einem der Ansprüche 1 bis 13 oder ein Set zum Konfigurieren der Gelenkanordnung nach Anspruch 14, wobei insbesondere die zumindest eine Gelenkanordnung (10) an einer lateralen oder einer medialen Seite des Extremitätengelenks angeordnet ist.
